# EUROPEAN PATENT SPECIFICATION

(11) **EP 4 208 146 B1**
(45) Date of publication and mention of the grant of the patent: **08.04.2026**
(21) Application number: 21863820.3
(22) Date of filing: 03.09.2021
(51) Int. Cl.: A61K 8/37, A61Q 19/00, A61K 8/31, A61K 8/49, A61K 8/64, A61K 8/92, A61K 8/97

(54) **NON-AQUEOUS COSMETIC COMPOSITION**
NICHTWÄSSRIGE KOSMETISCHE ZUSAMMENSETZUNG
COMPOSITION COSMÉTIQUE NON AQUEUSE

(30) Priority: 03.09.2020 IN 202031038030
(43) Date of publication of application: 12.07.2023
(73) Proprietor: Almora Botanica UK Ltd, London W1S 4LW (GB)
(72) Inventor: PATEL, Neeraj Kumar, Bidere Agarahara 560067 (IN); ROUT, Deeleep Kumar, Bangalore 560067 (IN)
(74) Representative: Buzzi, Notaro & Antonielli d'Oulx S.p.A.
(86) International application number: PCT/IB2021/058057
(87) International publication number: WO 2022/049532

(56) References cited:
- WO-A1-2010/115973
- US-A1- 2017 087 199
- DATABASE GNPD [online] MINTEL; 23 November 2018 (2018-11-23), ANONYMOUS: "The Dry Body Oil", XP093189124, retrieved from https://www.gnpd.com/sinatra/recordpage/6162537/ Database accession no. 6162537
- TAMAR GUTFINGER ET AL: "Studies of unsaponifiables in several vegetable oils", LIPIDS, SPRINGER-VERLAG, BERLIN/HEIDELBERG, vol. 9, no. 9, 1 September 1974 (1974-09-01), pages 658 - 663, XP035174105, ISSN: 1558-9307, DOI: 10.1007/BF02532171
- WOODRUFF JOHN: "Silicones and alternatives 2017 ", SPC 2017, 1 January 2017 (2017-01-01), pages 1 - 6, XP093037934
- WATSON KATHRYN, 21 February 2019 (2019-02-21), XP055913452, Retrieved from the Internet <URL:https://www.healthline.com/health/caprylic-capric-triglyceride>
- ANONYMOUS: "Caprylic/capric triglyceride", 17 October 2012 (2012-10-17), pages 1 - 2, XP093037937, Retrieved from the Internet <URL:http://www.saapedia.org/en/saa/?type=detail&id=2438> [retrieved on 20230406]

## Description

### FIELD OF THE INVENTION

The present invention relates to a field of non-aqueous cosmetic compositions. It further relates to liquid compositions that are free of surfactants and silicones and are substantially transparent.

### BAKGROUND AND PRIOR ART

Cosmetic compositions are formulated with skin care actives that provide various functional benefits. Many of these actives have relatively low solubility in water as well as oils, and are difficult to formulate as such formulation leads to precipitation or phase separation of actives, which is undesirable. Furthermore, such problems of phase separation become particularly severe at lower temperatures, particularly at temperatures less than 25 degree Celsius. Such compositions are formulated using various means to help in either solubilizing or dispersing such actives. Ingredients that aid solubilization or dispersion include surfactants, silicones, and other ingredients. There are concerns with respect to environmental sustainability and/or harmful effects on skin due to such ingredients.

WO2017/163182 A2 (2017) describes oily mixture for topical use comprising a first component and a second component, wherein said second component comprises at least one liposoluble component, characterized in that said first component comprises only an alkane and/or a precursor of said alkane, wherein said alkane is an alkane with a number of carbon atoms equal to 14 and/or wherein said precursor of said alkane comprises a number of carbon atoms of between C(10) and C(22), and in that said alkane and/or said precursor is of plant origin.

GB2566353 A (2019) describes a hair cleansing products containing anionic surfactants, amphoteric surfactants, a mixture of specific linear alkanes (C9 to C12 alkanes, especially dodecane, mixed with C15 to C23 alkanes, specifically docosane), and a non-polymeric structuring agent such as glycerides or castor oil, and are intended to be suitable for the cleansing and nourishing of hair, in particular in order to improve the wet and dry combability, the detangling capability, the feel, and the shine of hair.

WO2012/035065 (2012) describes cosmetic composition comprising at least one volatile linear alkane, a fatty ester of fatty alcohol and at least one plant oil, the esters being different from plant oils. It relates to the use of this composition for the cosmetic treatment of keratin materials, preferably keratin fibres such as the hair. WO2010115973 (2010) pertains to the field of cosmetic uses of said oily composition, particularly for makeup and/or care for skin, lips, eyelashes and/or nails, and describes a volatile oily composition comprising and preferably consisting of:
(a) from 50 to 100% by weight of a mixture of linear paraffins consisting of:
   (i) 70 to 99% by weight of at least one linear paraffin selected from C9, C10, C12 paraffins and mixtures thereof,
   (ii) 1 to 30% by weight of at least one C14 to C24 linear paraffin, and
(b) from 0 to 50% by weight of at least one non-volatile oil.

WO2019207527 (2017) describes a nonaqueous, silicone-free personal care composition comprising: 2 to 90%, preferably 10 to 70%, more preferably 10 to 50% by weight of a dialkyl carbonate, based on the total weight of the composition; and 2 to 95%, preferably 10 to 90%, more preferably 50 to 90% by weight of a C8-40 paraffin compound, based on the total weight of the composition, wherein the dialkyl carbonate is a bis(C1-36 alkyl)carbonate, preferably a bis(C1-24 alkyl)carbonate, more preferably a bis(C1-12 alkyl)carbonate, even more preferably a bis(C1-8 alkyl)carbonate that is manufactured by reacting urea and a C1-36 alcohol, preferably a C1-24 alcohol, more preferably a C1-12 alcohol, even more preferably a C1-8 alcohol.

Document "MINTEL; (2018-11-23), "The Dry Body Oil", database accession no. 6162537 (www.gnpd.com)" discloses a body oil that is non-aqueous, silicone and surfactant free and comprises C15-19 alkane, isoamyl cocoate, squalene and tocopherol.

### OBJECTS OF THE INVENTION

Silicones are well known for providing good spreading. However, there are concerns with respect of environmental impact as well as safety of silicones. Accordingly, one of the objects of the invention is to provide a non-aqueous cosmetic composition that is free of silicones.

In non-aqueous compositions, it is particularly difficult to incorporate active skin care ingredients without causing solubility problems or phase separation. Accordingly, another object of the present invention is to provide non-aqueous cosmetic composition comprising skin care active ingredients, where more amount of active can be solubilized at a given temperature, or alternately provide compositions with similar amount of active, but that will be stable at lower temperature, without phase separation of actives.

Surfactants also help in spreading and impart other useful properties. However, surfactants cause irritation to skin. Therefore, a further object of the present invention is to provide surfactant free cosmetic compositions.

Optical clarity or transparency is an important consumer preferred attribute. Accordingly, yet further objective of the present invention is to provide a cosmetic composition that is substantially clear or transparent.

A further object of the present invention is to provide cosmetic compositions having good spreadability and low viscosity for good sensory and in-use properties that are known to be preferred by consumers.

### SUMMARY OF THE INVENTION

The invention is defined by the claims. Any subject-matter falling outside the scope of the claims is provided for information purposes only.

According to the present description, there is provided a non-aqueous cosmetic composition comprising:
(A) 25-55% by weight C15-19 alkane;
(B) 15-30 % by weight C9-13 alkane;
(C) One or both of
   (i) 1-10% by weight isoamyl cocoate, or;
   (ii) 1-25% by weight cetyl ricinoleate, and;
(D) an active, wherein the weight ratio of the active to the rest of the composition is in the range 0.02:100 to 15:100, and the active is selected from:
   (i) extract of *Bidens pilosa;*

### DETAILED DESCRIPTION OF THE INVENTION

According to the present invention, there is provided a non-aqueous cosmetic composition as defined in the claims.

The term "non-aqueous" as used herein means a composition where water is less than 0.5% by weight of the composition, preferably less than 0.2% by weight of the composition and more preferably less than 0.05% by weight of the composition. Most preferably, water is less than 0.01% by weight of the composition.

It is further preferred that the non-aqueous cosmetic composition comprises both of the following:
(i) 1-10% by weight isoamyl cocoate, and;
(ii) 1-25% by weight cetyl ricinoleate

According to a further preferred aspect, the non-aqueous cosmetic composition comprises 1-25% by weight dicaprylyl carbonate.

Preferably, the non-aqueous cosmetic composition comprises one or both of the following:
1-25% by weight coco caprylate, or;
1-25% by weight capric/caprylic triglyceride.

More preferably, the non-aqueous cosmetic composition comprises both of the following:
1-25% by weight coco caprylate, and;
1-25% by weight capric/caprylic triglyceride.

It is further preferred that the the mixture of coco caprylate and capric caprylic triglyceride is 1-25% by weight of the composition.

It is further preferred that non-aqueous cosmetic composition comprises: 1-25% by weight coco caprylate, and 1-25% by weight capric caprylic triglyceride, and 1-25% by weight dicaprylyl carbonate, and 1-25% by weight of composition cetyl ricinoleate. More preferably, coco caprylate, capric caprylic triglyceride, dicaprylly carbonate and cetyl ricinoleate, all taken together are preferably present at 2-50% by weight of the composition, more preferably present at 3-40% by weight of the composition, and most preferably present at 5-30% by weight of the composition.

According to a preferred aspect, the non-aqueous cosmetic composition is free of surfactant. The term "free of surfactant" as used herein means a composition where surfactant is less than 0.5% by weight of the composition, preferably less than 0.2% by weight of the composition and more preferably less than 0.05% by weight of the composition. Most preferably, surfactant is less than 0.01% by weight of the composition.

The term "surfactant" as used herein means a substance which meets one of the following criteira: (a) 0.1% by weight of aqueous solution of the substance has air-water surface tension less than 40 mN/m, or (b) 0.1% by weight of non-aqueous solution of the substance in hexane has surface tension less than 15 mN/m.

It is preferred the the non-aqueous cosmetic composition is free of silicones. The term "free of silicones" as used herein means a composition where silicone is less than 0.5% by weight of the composition, preferably less than 0.2% by weight of the composition and more preferably less than 0.05% by weight of the composition. Most preferably, silicone is less than 0.01% by weight of the composition.

The non-aqueous cosmetic composition is preferably not solid. It is liquid at temperature preferably greater than or equal to 20 degrees Celsius.

According to a preferred aspect, the temperature at which the active solidifies (as seen by phase separation) is less than 20 degree Celsius, more preferably less than 17 degree Celsius, further preferably less than 15 degree Celsius, and even more preferably less than 10 degrees Celsius. It is particularly preferred that the temperature at which the phase separation of active occurs is less than 5 degrees Celsius, or even more preferably less than 2 degree Celsius.

It is preferred that the viscosity of the non-aqueous cosmetic composition at 25 degrees Celsius measured at shear rate of 21.2 per second is less than 10 mPa.S, more preferably less than 7 mPa.s, and most preferably less than 5 mPa.s. The viscosity of the non-aqueous compositon is preferably greater than 0.1 mPa.s

It is preferred that the spreading coefficient of the non-aqueous cosmetic composition at 25 degrees Celsius is greater than 400 mm squared per 10 minutes. The spreading coefficient at 25 deg Celsius is preferably greater than 450 mm squared per 10 minutes, and more preferably greater than 500 mm squared per 10 minutes. In the most preferred aspect, the spreading coeffieicent of the non-aqueous cosmetic composition is greater than 600 mm squared per 10 minutes. The spreading coefficient of the non-aqueous cosmetic composition is preferably less than 2000 mm squared per 10 minutes

It is preferred that visible light transmittance (%T) of the non-aqueous cosmetic composition is greater than 80%. The transmittance (%T) is preferably greater than 85%, more preferably greater than 87% and most preferably greater than 90%. The %T measurements can be carried out by an UV-VIS spectrophotometer (wavelength region 400-700 nm) with a cell of cell length 10 mm.

C15-19 Alkane is a mixture of alkanes with 15 to 19 carbon atoms in the alkyl chain. Examples of C15-19 alkane include, but are not limited to Emogreen L19^{®} (Seppic) Emosmart L19^{®} (Seppic) Gemseal 40^{®} (Total Special Fluids). C9-13 Alkane refers to a mixture of alkanes with 9 to 13 carbon atoms in the alkyl chain. Preferably it is a mixture of undecane (C9) and tridecane (C13). Examples of C9-13 alkane include, but are not limited to Cetiol^{®} Ultimate (BASF).

Examples of isoamyl cocoate available commercially include but are not limited to Tegosoft AC^{®} (Evonik) and Oxismmoth Co^{®} (Oxiteno). The non-aqueous cosmetic composition comprises preferably 1-10% by weight, more preferably 1.5-8% by weight, and most preferably 2-7% by weight isoamyl cocoate. Examples of cetyl ricinoleate available commercially include, but are not limited to Tegosoft CR ^{®} (Evonik) Sabowax CR^{®} (Sabo) DUB Rc ^{®} (Stearineriie Dubois) Jeechem CER ^{®} (Jeen Intl) Demol CTR ^{®} (Alzo) Protachem CER ^{®} (Protameen) Ambuchem CR ^{®} (Ambuja Solvex). The non-aqueous cosmetic composition comprises preferably 1-25% by weight, more preferably 5-20% by weight, and most preferably 8-15% by weight cetyl ricinoleate.

Examples of coco caprylate available commercially include, but are not limited to, Cetiol^{®} C5 (BASF) Ercarel^{®} (Ercawilmer) Domusare^{®} (Domus Chemicals) Rotefan^{®} (Ecogreen Oleo) Cremecoor Coco810^{®} (cremer Oleo) Dub 810C ^{®} (Stearinierie Dubois) Massocare^{®} (Commmercial Chimica Masso) Lanol 2681^{®} (Seppic) Miglyol 810 ^{®} (IOI Oleo) Captex 170 ^{®} (Abitech). The non-aqueous cosmetic composition comprises preferably 1-25% by weight, more preferably 2-20% by weight, and most preferably 5-15% by weight coco caprylate.

Examples of capric caprylic triglyceride available commercially include, but are not limited to Radia^{®} 7104 (Seppic), Velsan^{®} (Clariant) Silk SC ^{®} (Zchimmer & Schwartz), Emarol^{®} 3139 (Chemir) Palmster^{®} (KLK Oleo) Myritol^{®} (BASF) GalMOL^{®} (Galaxy). The non-aqueous cosmetic composition comprises preferably 1-25% by weight, more preferably 10-24% by weight, and most preferably 15-22% by weight capric caprylic triglyceride.

Examples of dicaprylyl carbonate available commercially include but are not limited to Cetiol CC ^{®} (BASF) Lonzest DC NT^{®} (Lonza). The non-aqueous cosmetic composition comprises preferably 1-25% by weight, more preferably 2-20% by weight, and most preferably 5-15% by weight dicaprylyl carbonate.

The non-aqueous cosmetic composition comprises an active wherein the weight ratio of the active to the rest of the composition is in the range 0.02:100 to 15:100, and the active is an
extract of *Bidens pilosa* .

The weight ratio of the active to the rest of the composition is preferably in the range 0.02:100 to 15:100, more preferably in the range of 0.05:100 to 13:100, and further preferably in the range 0.1:100 to 12:100, and most preferably in the range 0.2:100 to 11:100.

The active is preferably an extract of flowers or fruits of *Bidens pilosa.* The extraction is preferably carried out using supercritical fluid extraction, preferably supercritical carbon dioxide extraction. It may be used as an active for its properties which include, but are not limited to skin-firming, lightening, anti-aging, antioxidant, skin hydration and anti-wrinkle. *Bidens pilosa* extract has a rich phytochemical composition, standardized in phytol. *Bidens Pilosa* extract is a BioRetinol with retinoids receptors activity, mimicking the same positive results that retinoids cause to fight against skin aging. Skin visibly look younger, luminous and firm, reducing wrinkles, improves skin elasticity and ECM redensification, once stimulates the synthesis of dermic proteins and growth factors. Revinage^{®} increases ceramide 3 and NMF in the lips, improving protection and hydration. An example of commercially available active is Revinage^{®} (Chemyunion, Brazil) which comprises 12-28% of extract of *Bidens pilosa,* oils and tocopherol.

### EXAMPLES

The invention shall be demonstrated with the examples. It should be appreciated that the examples are for the illustration purpose only and do not in any way limit the scope of the invention.

Preparation of compositions: Following procedure was followed:
All ingredients were weighed and added to a glass beaker (1 L capacity) and mixed using overhead stirrer (IKA Eurostar 20 digital, 200 rpm for 10 min). The mix was heated on a hot plate to 60 °C (± 5 °C) till all the ingredients dissolve. The compositions were cooled to 25 deg C and transferred to containers for storage and subsequent measurements.

### Measurement of viscosity of compositions:

To measure the viscosity, an Anton Paar MCR 92 rheometer was used. The following settings were used in the RheoCompass software:
No. of datapoints: 50
Shear rate at which the viscosity value was observed: 21.2 s⁻¹
Time interval between data points: 6 secs
Total time: 300 seconds
Starting shear rate: 0.1 s⁻¹

### Measurement of spreading coefficient of compositions:

To measure the spreading coefficient, ashless Whatman filter papers (1, 125 mm dia, Cat. No. 1001125) (medium-fast retention) were used. A 20 µL sample of the composition was taken and added to the centre of the filter paper. After 10 minutes, the area within which the oil spreads is measured. Typically the composition spreads as a circle and its diameter is measured to obtain the area of the circle obtained. The spreading coefficient is expressed as mm squared per 10 minutes.

### Measurement of % transmittance:

The %T measurements are carried out by an UV-VIS spectrophotometer (wavelength region 400-700 nm) with a cell of cell length 10 mm.

### Measurement of temperature stability of the compositions comprising actives:

Following procedure was followed. The active was solubilised in the composition at an elevated temperature (45 C) by magnetic stirring to ensure a complete solubilisation and equilibrated overnight at 45 C using a stability chamber. The composition comprising the active was cooled down at a given rate (3.5 deg C/min) and the active precipitation was detected by measuring the storage and loss modulii at 1 Hz frequency using an Anton Paar MCR 92 rheometer. The following settings were used in the RheoCompass software:
0.1 % strain
No. of datapoints: 100
Time interval between data points: 6 secs
Total time: 600 seconds
Starting Temperature: 30 C.

The precipitation temperature of the active was determined by noting the temperature at which storage modulus becomes larger than the loss modulus. This precipitation temperature is a direct measure of temperature at which phase separation of active will be observed. Higher temperature of phase separation of active is indicative if lower stability of the composition with respect to temperature, and lower temperature of phase separation of active is indicative of higher stability of the composition with respect to temperature.

### Examples 1-3:

Compositions of Examples 1-3 were prepared according to the procedure described earlier. The details of the ingredients used are given below:
C15-19 alkane (Emogreen L19) was procured from SEPPIC, France.
C9-13 alkane (Undecane+Tridecane) was procured from BASF, India.
Isoamyl cocoate (Tegosoft AC) was procured from Evonik India.
Capric caprylic triglyceride (Radia 7104) was procured from Evonik India.
Coco caprylate (Cetiol C5) was procured from BASF India.
Dicaprylyl carbonate (Cetiol CC) was procured from BASF, India.
Cetyl ricinoleate (Tegosoft CR) was procured from Evonik, India.
The active used was an extract of *Bidens pilosa* that is commercially available as Revinage^{®} . The extract of Bidens pilosa (Revinage^{®},) was procured from Chemyunion, Brazil. It has a melting temperature of 67.5 degree Celsius.

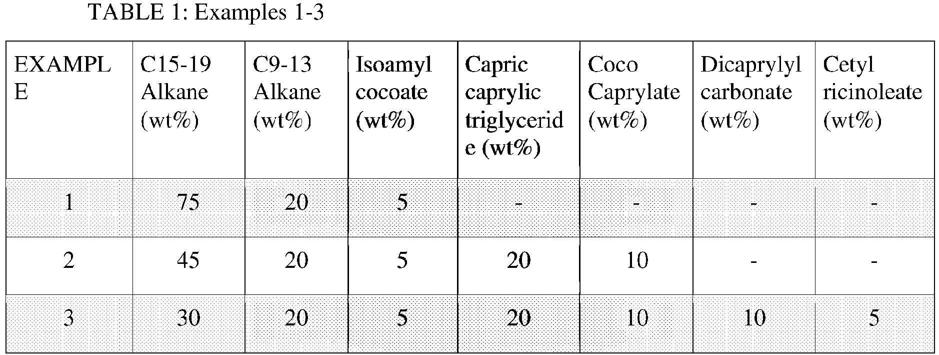

Compositions of Examples 1-3 were prepared according the procedure described earlier. Example 1 is not according to the claimed invention.

In the compositions of Examples 1-3, active Revinage^{®} *(Bidens pilosa* extract) was added such that weight ratio of the active to the rest of the ingredients in the composition was 5:100, and the precipitation temperature was determined according to the protocol described for the measurement of the solubility of the active. The results are tabulated below:

**TABLE 2: Precipitation temperature for Examples 1-3 with weight ratio of active (Revinage^{®} to the rest of the composition = 5:100)**

| **Example** | **Precipitation Temperature (deg. Celcius) or Phase separation temperature** |
|---|---|
| 1 | 17 |
| 2 | 15 |
| 3 | 7 |

All the compositions (EXAMPLES 1,2,3) exhibit acceptable precipitation temperature.As seen, the composition of Example 3 has relatively lower precipitation temperature than the compositions of Examples 1 and 2.

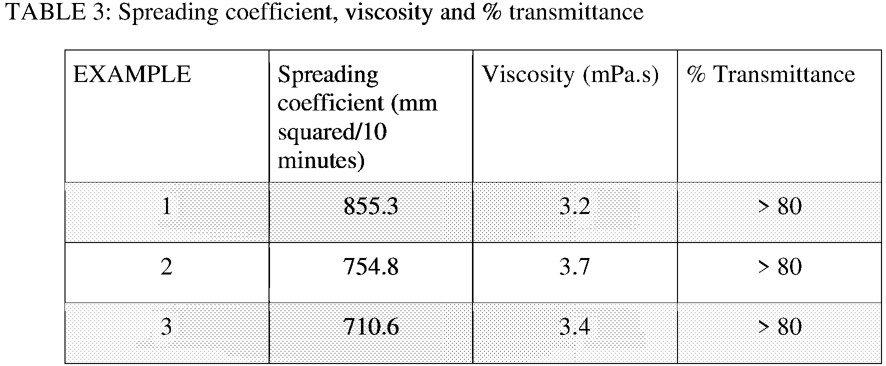

The Table above indicates the values of spreading coefficient, viscosity, and % transmittance of the compositions of Examples 1-3 to which active Revinage^{®} has been added such that the weight ratio of the active to the rest of the composition is 5:100.

It can be seen that spreading coefficient of the compositions of Examples 2-3 are above 700 mm squared per 10 minutes, thus capable of providing adequate spreading performance. Furthermore, compositions of Examples 2-3 is below 4 mPa.s, and % transmittance is greater than 80%. Thus the compositions of Examples 1 and 2 have better ability to solubilise active ingredient, whilst providing adequate performance with respect to viscosity, spreading and % transmittance.

**TABLE 4: EXAMPLES 4-15**

| EXAMPL E | C15-19 Alkane (wt%) | C9-13 Alkane (wt%) | Isoamyl cocoate (wt%) | Capric caprylic triglycerid e (wt%) | Coco Caprylate (wt%) | Dicaprylyl carbonate (wt%) | Cetyl ricinoleate (wt%) |
|---|---|---|---|---|---|---|---|
| 4 | 75 | 20 | - | - | - | - | 5 |
| 5 | 45 | 20 | - | 20 | 10 | - | 5 |
| 6 | 50 | 33.4 | 8.3 | 8.3 | 0 | 0 | 8.3 |
| 7 | 42.8 | 28.5 | 7.1 | 0 | 0 | 0 | 7.1 |
| 8 | 55 | 20 | 0 | 20 | 0 | 0 | 5 |
| 9 | 65 | 20 | 0 | 0 | 10 | 0 | 5 |
| 10 | 35 | 20 | 0 | 20 | 10 | 10 | 5 |
| 11 | 40 | 20 | 5 | 20 | 10 | 0 | 5 |
| 12 | 65 | 20 | 0 | 0 | 0 | 10 | 5 |
| 13 | 70 | 20 | 5 | 0 | 0 | 0 | 5 |
| 14 | 60 | 20 | 5 | 0 | 0 | 10 | 5 |
| 15 | 70 | 20 | 0 | 0 | 0 | 0 | 10 |

Compositions of Examples 4-15 were prepared according the procedure described earlier. Examples 4, 6, 9 and 12-15 do not represent the claimed invention.

In the compositions of Examples 4-15, active Revinage^{®} (Bidens pilosa extract) was added such that weight ratio of the active to the rest of the ingredients in the composition was 10:100. Various parameters of the resulting compositions were measured as per the protocols described earler result are tabulated below:

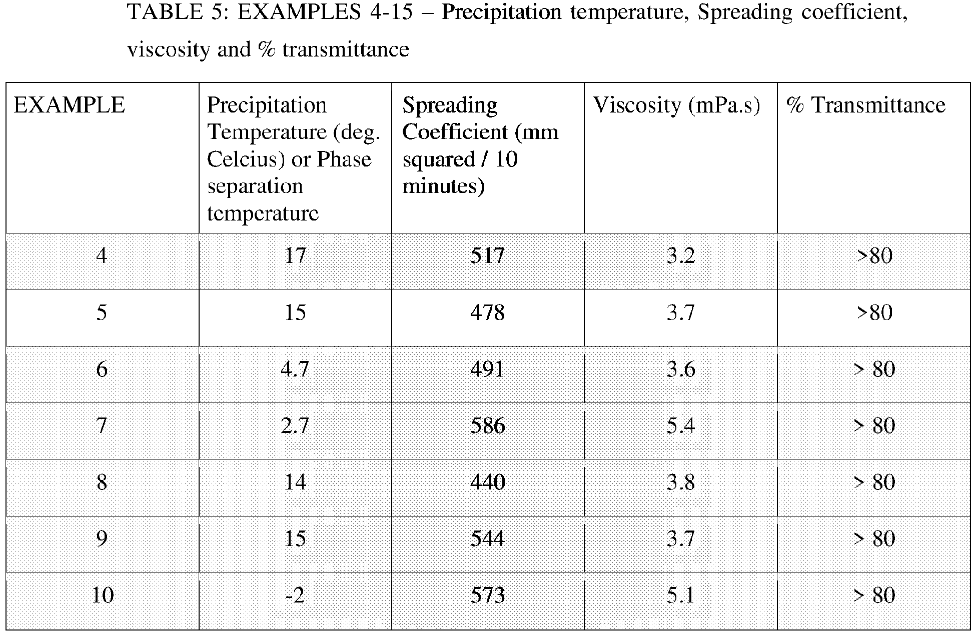

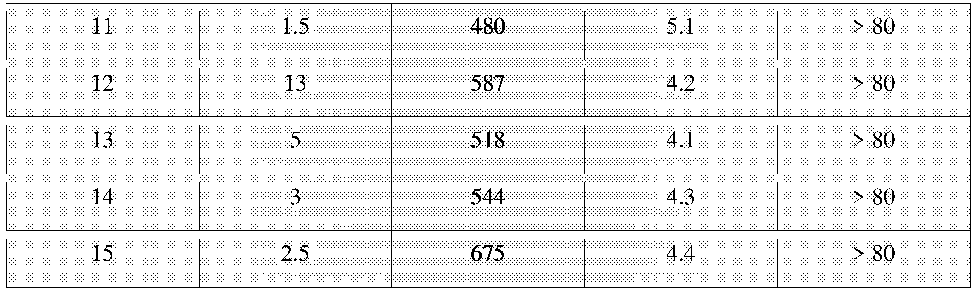

It can be seen that all the compositions above (EXAMPLES 4-15), exhibit acceptable temperature stability, spreading coefficient and viscosity and % transmittance. The compositions with lower solidifaction temperature are preferred over those with higher solidification temperature. The compositions with higher spreading coefficient are preferred over those with lower spreading coefficient.

It can be seen from the foregoing examples that the non-aqueous cosmetic composition of the present invention meets one or more of the objects of the invention.

## Claims

1. A non-aqueous cosmetic composition comprising:
(A) 25-55% by weight C15-19 alkane;
(B) 15-30% by weight C9-13 alkane;
(C) One or both of
(i) 1-10% by weight isoamyl cocoate, or;
(ii) 1-25% by weight cetyl ricinoleate, and;
(D) an active, wherein the weight ratio of the active to the rest of the composition is in the range 0.02:100 to 15:100, and wherein the active is extract of *Bidens pilosa,*
wherein the composition comprises less than 0.5% of water by weight.

2. A non-aqueous cosmetic composition as claimed in claim 1 comprising: 1-10% by weight isoamyl cocoate, and 1-25% by weight cetyl ricinoleate

3. A non-aqueous cosmetic composition as claimed in claim 1 or claim 2 comprising 1-25% by weight dicaprylyl carbonate.

4. A non-aqueous cosmetic composition as claimed in any one of the preceding claims comprising one or both of the following:
1-25% by weight coco caprylate, or;
1-25% by weight capric/caprylic triglyceride.

5. A non-aqueous cosmetic composition as claimed in claim 4 comprising 1-25% by weight coco caprylate and 1-25% by weight capric/caprylic triglyceride.

6. A non-aqueous cosmetic composition as claimed in any one of the preceding claims wherein the composition is free of surfactant such that it comprises less than 0.5% by weight of surfactant in the composition.

7. A non-aqueous cosmetic composition as claimed in any one of the preceding claims wherein the composition is liquid at temperature greater than or equal to 20 degree Celsius.

8. A non-aqueous cosmetic composition as claimed in any one of the preceding claims wherein the composition is free of silicones such that it comprises less than 0.5% by weight of silicone in the composition.

9. A non-aqueous cosmetic composition as claimed in any one of the preceding claims wherein the viscosity of the composition at 25 degree Celsius measured at shear rate of 21.2 per second is less than 10 mPa.S measured using Anton Paar MCR 92 Viscometer.

10. A non-aqueous cosmetic composition as claimed in any one of the preceding claims wherein the spreading coefficient of the composition at 25 degree Celsius is greater than 400 mm squared per 10 minutes measured using ashless Whatman filter papers (1, 125 mm dia, Cat. No. 1001125).

## Patentansprüche

1. Nichtwässrige kosmetische Zusammensetzung, umfassend:
(A) 25-55 Gew.-% C15-19-Alkan;
(B) 15-30 Gew.-% C9-13-Alkan;
(C) eines oder beide von
(i) 1-10 Gew.-% Isoamylcocoat oder;
(ii) 1 bis 25 Gew.-% Cetylricinoleat, und;
(D) einem Wirkstoff, wobei das Gewichtsverhältnis des Wirkstoffs zum Rest der Zusammensetzung im Bereich von 0,02:100 bis 15:100 liegt und wobei der Wirkstoff ein Extrakt aus *Bidens pilosa* ist,
wobei die Zusammensetzung weniger als 0,5 Gew.-% Wasser enthält.

2. Eine nichtwässrige kosmetische Zusammensetzung gemäß Anspruch 1, umfassend: 1-10 Gew.-% Isoamylcocoat und 1-25 Gew.-% Cetylricinoleat.

3. Eine nichtwässrige kosmetische Zusammensetzung gemäß Anspruch 1 oder Anspruch 2, umfassend 1-25 Gew.-% Dicaprylylcarbonat.

4. Nichtwässrige kosmetische Zusammensetzung nach einem der vorstehenden Ansprüche, umfassend eines oder beide der folgenden:
1 bis 25 Gew.-% Coco-Caprylat oder
1 bis 25 Gew.-% Capric/Caprylic-Triglycerid.

5. Nichtwässrige kosmetische Zusammensetzung nach Anspruch 4, umfassend 1 bis 25 Gew.-% Coco-Caprylat und 1 bis 25 Gew.-% Capric/Caprylic-Triglycerid.

6. Nichtwässrige kosmetische Zusammensetzung nach einem der vorstehenden Ansprüche, wobei die Zusammensetzung frei von Tensiden ist, sodass sie weniger als 0,5 Gew.-% Tenside in der Zusammensetzung umfasst.

7. Nichtwässrige kosmetische Zusammensetzung nach einem der vorstehenden Ansprüche, wobei die Zusammensetzung bei einer Temperatur von größer oder gleich 20 Grad Celsius flüssig ist.

8. Nichtwässrige kosmetische Zusammensetzung nach einem der vorstehenden Ansprüche, wobei die Zusammensetzung frei von Silikonen ist, so dass sie weniger als 0,5 Gew.-% Silikon in der Zusammensetzung umfasst.

9. Nichtwässrige kosmetische Zusammensetzung nach einem der vorstehenden Ansprüche, wobei die Viskosität der Zusammensetzung bei 25 °C, gemessen bei einer Schergeschwindigkeit von 21,2 pro Sekunde, weniger als 10 mPa·s beträgt, gemessen mit einem Anton Paar MCR 92 Viskosimeter.

10. Nichtwässrige kosmetische Zusammensetzung nach einem der vorstehenden Ansprüche, wobei der Ausbreitungskoeffizient der Zusammensetzung bei 25 °C größer als 400 mm² pro 10 Minuten ist, gemessen unter Verwendung von aschefreien Whatman-Filterpapieren (1, 125 mm Durchmesser, Kat.-Nr. 1001125).

## Revendications

1. Composition cosmétique non aqueuse comprenant :
(A) 25 à 55 % en poids d'alcane en C₁₅ à C₁₉ ;
(B) 15 à 30 % en poids d'alcane en C₉ à C₁₃ ;
(C) un ou les deux parmi
(i) 1 à 10 % en poids de coprah-ylate d'isoamyle ; et
(ii) 1 à 25 % en poids de ricinoléate de cétyle ; et
(D) un principe actif, dans lequel le rapport en poids du principe actif au reste de la composition est situé dans la plage allant de 0,02/100 à 15/100, et lequel principe actif est un extrait de *Bidens pilosa,*
laquelle composition comprend moins de 0,5 % en poids d'eau.

2. Composition cosmétique non aqueuse selon la revendication 1, comprenant 1 à 10 % en poids de coprah-ylate d'isoamyle et 1 à 25 % en poids de ricinoléate de cétyle.

3. Composition cosmétique non aqueuse selon la revendication 1 ou la revendication 2, comprenant 1 à 25 % en poids de carbonate de dicaprylyle.

4. Composition cosmétique non aqueuse selon l'une quelconque des revendications précédentes, comprenant un ou les deux parmi
1 à 25 % en poids de caprylate dérivé du coprah ; et
1 à 25 % en poids de triglycéride caprique/caprylique.

5. Composition cosmétique non aqueuse selon la revendication 4, comprenant 1 à 25 % en poids de caprylate dérivé du coprah et 1 à 25 % en poids de triglycéride caprique/caprylique.

6. Composition cosmétique non aqueuse selon l'une quelconque des revendications précédentes, laquelle composition est exempte de tensioactif de sorte qu'elle comprenne moins de 0,5 % en poids de tensioactif dans la composition.

7. Composition cosmétique non aqueuse selon l'une quelconque des revendications précédentes, laquelle composition est liquide à une température supérieure ou égale à 20 degrés Celsius.

8. Composition cosmétique non aqueuse selon l'une quelconque des revendications précédentes, laquelle composition est exempte de silicones de sorte qu'elle comprenne moins de 0,5 % en poids de silicone dans la composition.

9. Composition cosmétique non aqueuse selon l'une quelconque des revendications précédentes, dans laquelle la viscosité de la composition à 25 degrés Celsius, mesurée à une vitesse de cisaillement de 21,2 par seconde, est inférieure à 10 mPa.s, mesurée au moyen d'un viscosimètre Anton Paar MCR 92.

10. Composition cosmétique non aqueuse selon l'une quelconque des revendications précédentes, dans laquelle le coefficient d'étalement de la composition à 25 degrés Celsius est supérieur à 400 mm carrés par 10 minutes, mesuré au moyen de papiers filtres Whatman sans cendres (1, diamètre 125 mm, numéro de catalogue 1001125).
